# EUROPEAN PATENT APPLICATION

(11) **EP 4 378 923 A1**
(43) Date of publication of application: **05.06.2024**
(21) Application number: 22210855.7
(22) Date of filing: 01.12.2022
(51) Int. Cl.: C07C 231/02

(54) **PRODUCTION OF PANTHENOL**

(71) Applicant: DSM IP Assets B.V., 6411 TE Heerlen (NL)
(72) Inventor: STEMMLER, René Tobias, 4303 Kaiseraugst (CH); MEDLOCK, Jonathan Alan, 4303 Kaiseraugst (CH); HUNZIKER, Nadine, 4303 Kaiseraugst (CH); HOUSTON, Peter Louis, 4303 Kaiseraugst (CH)
(74) Representative: dsm-firmenich IP

(57) **Abstract**

The present invention provides a novel process for producing panthenol from pantothenic acid or salts and/or esters thereof. The process makes a "hybrid process" possible to produce panthenol, which is generally more eco-friendly and reduces carbon print compared to a pure chemical process.

## Description

The present invention relates to an improved process for producing panthenol from pantothenic acid or salts and/or esters thereof.

Panthenol is an alcohol derivative of pantothenic acid or vitamin B₅ which is commercially used in various cosmetic products. Conventional production of panthenol is through chemical synthesis by condensing 3-aminopropanol with pantolactone, which is produced in industry by several chemical steps and normally needs an additional resolution treatment to obtain the desired (*R*)-pantolactone.

Over the years, several microbial fermentation processes were developed aiming to replace the existing chemical processes. Nowadays use of the genetically engineered strain *Bacillus subtilis* represents the most efficient process for producing pantothenic acid and salts thereof by fermentation. (see WO0121772 and WO02057474) However, there is still no report on a fermentation processes for producing panthenol directly.

Accordingly, there is an ongoing demand in industry to effectively convert fermentatively produced pantothenic acid into highly pure panthenol.

Hence, the present invention provides a new process for producing panthenol from pantothenic acid or salts and/or esters thereof, said process comprising the conversion of pantothenic acid, or salts and/or esters thereof, particularly fermentatively produced pantothenic acid or salts thereof, into panthenol. As said process can use biotechnologically produced compounds including but not limited to pantothenic acid or salts and/or esters thereof, said route furthermore can satisfy the need for production of eco-friendly, sustainable and carbon neutral products.

Particularly, the present invention provides a process for producing a compound of formula (I), comprising the steps of:
a)treating a compound of formula (II) with an acid to obtain pantolactone; and 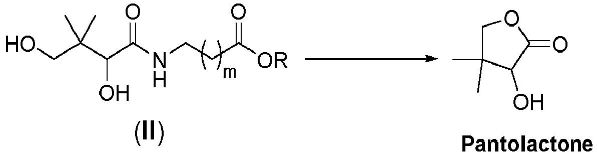
b) reacting the pantolactone with a compound of formula (III) to obtain the compound of formula (I), 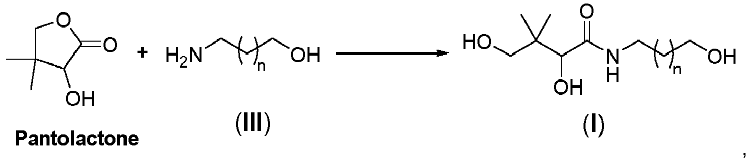
wherein R is selected from the group consisting of M or hydrogen or substituted or unsubstituted alkyl, wherein M is an alkali metal or earth alkali metal, particularly sodium, potassium, calcium, or magnesium, and each of m and n is independently an integer between 0-5.

Preferably, the substituted or unsubstituted alkyl is selected from substituted or unsubstituted C₁-C₁₀ alkyl, such as e.g. C₁, C₂, C₃, C₄, C₅, C₆, C₇, C₈, C₉ or C₁₀-alkyl, more preferably selected from methyl, ethyl, n-propyl, iso-propyl, n-butyl, iso-butyl, tert-butyl, or benzyl.

Preferably, each of m and n is independently 1, 2 or 3, and more preferably, m and n are independently 1.

In the present invention, the compound of formula (II) is preferably selected from pantothenic acid (R = H), a salt of pantothenic acid (M is an alkali metal or earth alkali metal), such as calcium-pantothenate, sodium-pantothenate, magnesium-pantothenate and potassium-pantothenate, or pantothenic acid esters (R = substituted or unsubstituted alkyl) including but not limited to pantothenic acid methyl ester or pantothenic acid ethyl ester, wherein these compounds can either be produced by a chemical process as e.g. described in Martin et al. (J. Am. Chem. Soc., Vol. 116, No. 11, 1994) or in WO2017099822, or by a biocatalytic process or preferably via a fermentative process as e.g. described in WO0121772 or WO02057474, including esterification of fermentatively produced pantothenic acid.

It is well understood that in case of M being earth alkali metal, the respective ions are divalent and, hence, formally such metals in the formula (II) would be ½, e.g. ½ Ca, ½ Mg.

In the present invention, the compound of formula (II) is preferably pantothenic acid or a salt thereof, more preferably pantothenic acid or the calcium salt thereof.

In the present invention, the compound of formula (II) is preferably fermentatively produced.

In the present invention, the compound of formula (III) is preferably 3-aminopropanol.

In the present invention, the compound of formula (I) is preferably panthenol.

In the present invention, the compound of formula (II), pantolactone or the compound of formula (I) may be present in any configuration, such as e.g. in the (*R*) or (*S*)-configuration or occurring as racemate in (*R*/*S*)-configuration, or an enantiomerically enriched mixture where one isomer is in excess, whereby the (*R*) or (*R*/*S*)-configuration is preferred. Typically, if the compound of formula (II) is produced by a fermentation process, a percentage of at least about 95%, such as e.g. about 97%, 98%, 99% or even 100% of (*R*)-configuration based on total weight of the compound of formula (II) is given.

In the step a) of the process of the present invention, the acid may be selected from any one or more of organic acids, inorganic acids and/or acidic cation exchange resins. Examples of the organic acids include but are not limited to carboxylic acids such as formic acid, acetic acid and trifluoroacetic acid, and sulfonic acid or derivatives thereof such as methanesulfonic acid, tosic acid and trifluoromethanesulfonic acid. Examples of the inorganic acids include but are not limited to hydrochloric acid, sulfuric acid, -phosphoric acid and nitric acid. Examples of the acidic cation exchange resins include but are not limited to polymeric organosulfonic acids such as Amberlyst 15 (available from Dow Chemical).

In the step a) of the process of the present invention, the acid is preferably selected from the group consisting of formic acid, acetic acid, trifluoroacetic acid, hydrochloric acid, sulfuric acid, sulfonic acid, methanesulfonic acid, tosic acid, trifluoromethanesulfonic acid, phosphoric acid, nitric acid and acidic cation exchange resins such as Amberlyst 15. More preferably, the acid is trifluoroacetic acid, hydrochloric acid, sulfuric acid, methanesulfonic acid, trifluoromethanesulfonic acid, phosphoric acid, nitric acid and Amberlyst 15. Even more t preferably, the acid is selected from the group consisting of hydrochloric acid, sulfuric acid, methanesulfonic acid, nitric acid and Amberlyst 15. The most preferred acids in step a) are methanesulfonic acid and sulfuric acid, as these acids lead to particularly good yields and/ or purities.

In the step a) of the process of the present invention, the acid may be added in an amount of from 0.01 mol to 20 moles, preferably from 0.1 moles to 10 moles, more preferably from 1 moles to 8 moles such as 1, 2, 3, 4, 5, 6 ,7 and 8 moles, per 1 mole of the compound of formula (II). Further suitable ranges encompass from 2.5 to 10, from 3 to 10, from 4 to 10, from 2.5 to 7.5, from 3 to 6, and from 4 to 6 moles per mole of the compound of formula (II).

In the step a) of the process of the present invention, the acid is preferably used in an aqueous solution. The concentration of the acid in the aqueous solution may be from 0.1 M (mol/L) to 10 M, preferably from 0.1 M to 5 M, more preferably from 1 M to 3 M such as 2.5 M.

In the step a) of the process of the present invention, an additional solvent may be used but it is not preferable. The solvent may be a non-aqueous, organic, polar or non-polar solvent. Suitable solvents may be selected from alcohols and polyols, esters, ethers, amides, nitriles, hydrocarbons or chlorinated hydrocarbons with or without substitutions. Preferably, no additional solvent is used in the process of the present invention.

In the step a) of the process of the present invention, the reaction may be carried out at a temperature selected in the range of about 20°C to about 150°C, preferably of about 40°C to about 120°C such as 80°C, wherein pantolactone is produced.

The treatment in the step a) of the process of the present invention may be kept for 1-20 hours, preferably 2-16 hours. After the treatment, the obtained pantolactone can be used into the next step b) directly or can be separated from the by-products produced in the step a) before being used into the step b).

Preferably, the obtained pantolactone is separated from the by-products produced in the step a), which are mainly metal salts such as calcium salts and amino acids such as beta-alanine and/or salts thereof.

More preferably, the obtained pantolactone is separated from the by-products by extraction, and the extract or its concentrate containing pantolactone is used in the step b).The suitable extraction solvents are known in the art and the examples include but are not limited to esters and lactones such as ethyl acetate and isopropyl acetate, ketones such as methyl ethyl ketone, ethers such as diethyl ether and methyl tert-butyl ether, and hydrocarbons such as toluene and heptane.

In the present invention, the beta-alanine and/or salts thereof produced as a by-product of step a) may be further purified and used in other purpose.

In the step b) of the process of the present invention, the compound of formula (III) may be used in an amount of from 0.5 moles to 1.5 moles, preferably from 0.8 moles to 1.2 moles, per 1 mole of pantolactone.

The reaction in the step b) may be carried out in a solvent suitable for the reaction. Examples of the suitable solvent include but are not limited to any one or more alkyl alcohols such as methanol and ethanol. The solvent may be used in an amount of from 50 mL to 1000 mL, preferably from 100 mL to 300 mL such as 150 mL, per 1 mole of pantolactone used in the step b).

The reaction temperature of the step b) may be at from 10°C to 160°C, preferably at from 20°C to 100°C, more preferably from 40°C to 80°C.

The obtained compound of formula (I) can be purified (when needed) using commonly known methods, such as e.g. distillation as described in e.g. US20120149903.

Preferably, the present invention provides a process for producing panthenol, comprising the step **i**) of fermentative production of pantothenic acid/pantothenate optionally followed by esterification thereof into pantothenic acid esters, and the step **ii**) of treating the pantothenic acid or salts and/or esters thereof obtained in the step **i**) with an acid to obtain pantolactone, and the step **iii**) of reacting the pantolactone obtained in the step **ii**) with 3-aminopropanol to obtain panthenol, wherein the step **ii**) is carried out as described above about the step a) and the step **iii**) is carried out as described above about the step b).

Fermentative production of pantothenic acid is known in the art, see e.g. WO0121772 or WO02057474. The esterification can be done according to standard procedures in the art, including but not limited to biocatalytic or fermentative processes. Chemical esterification, e.g. by reacting pantothenic acid with the respective alcohol in the presence of an acid is however preferred. As used herein, such process combining chemical production steps and biotechnological steps is referred to as a "hybrid process".

Even more preferably, in all embodiments of the present invention, the use of pantothenic acid derived from microorganism metabolism of plant-derived sugars and alcohols composed of carbon of atmospheric origin, and not composed of fossil-fuel carbon is preferred. Said pantothenic acid has an anthropogenic CO₂ emission profile of zero upon biodegradation, which is particularly desired.

The process of the present invention makes a "hybrid process" possible to produce the compound of formula (I) such as panthenol, which is generally more eco-friendly and sustainable and reduces the carbon footprint compared to a pure chemical process using fuel based raw materials. In addition, the process of the present invention provides the compound of formula (I) such as panthenol preferably in (*R*)-configuration, wherein at least about 95% such as about 97, 98, 99 or even 100% of the compound of formula (I) is present as (*R*)-configuration based on the total weight of the compound of formula (I).

Furthermore, by removing the by-products such as amino acids obtained in the step a), it becomes easier to purify the obtained compound of formula (I) such as panthenol from the reaction mixture of the step b).

The invention is illustrated by the following Examples. All percentages in the examples are related to the weight.

### Example 1

Calcium (*R*)-pantothenate (24.3 g, 50.0 mmol, 98% purity) was dissolved in aqueous HCI (2.5 M, 100 mL, 250 mmol) and heated to 80°C for 2 h. After cooling the product mixture was extracted with ethyl acetate (4 × 250 mL), the combined organic phases were dried over MgSO₄ and concentrated *in vacuo* to obtain (R)-pantolactone (12.7 g, 99% yield, 99% purity by qNMR, >99.7% e.e. by Chiral HPLC (Chiralpak AS-H)).

### Example 2

Following the same procedures according to Example 1, different acids were used. The reaction conditions and results are shown in the below table.

| **En try** | **Acids** | | | **Conc. of SM (M)** | **Reaction Temperature (°C)** | **Reaction Time (h)** | **Yield (%)** | **Purity (%)** | **% e.e. of (*R*)-pantolactone** |
|---|---|---|---|---|---|---|---|---|---|
| | **Name** | **Conc. (wt%)** | **Mole eq.** | | | | | | |
| 1 | HCI | 17 | 10 | 0.5 | 80 | 16 | 85 | 98.8 | nd |
| 2 | H₂SO₄ | 12 | 5 | 0.25 | 80 | 2 | 86 | 99.9 | nd |
| 3 | Amberlyst 15 | - | 5 | 0.25 | 80 | 16 | 76 | 98.8 | 97.9 |
| 4 | MeSO₃H | 33 | 5 | 0.74 | 80 | 2 | 97 | 99.5 | nd |
| 5 | HNO₃ | 5 | 5 | 0.15 | 80 | 2 | 86 | 94 | nd |

| | | | | | | | | | |
|---|---|---|---|---|---|---|---|---|---|
| *SM =starting material; nd =not determined. | | | | | | | | | |

### Example 3

(*R*)-Pantolactone (3.94 g, 30.0 mmol, 99% purity) was dissolved in methanol (4.5 mL). 3-aminopropan-1-ol (2.28 g, 30.0 mmol, 99% purity) was added. The mixture was mixed thoroughly and then left to stand tightly closed for 24 h at room temperature. The resulting colorless, viscous solution was evaporated in vacuo to obtain (*R*)-pantothenol (6.15 g, 27.3 mmol, 91% purity by qNMR, 91% yield).

## Claims

1. A process for producing a compound of formula (I), comprising the steps of
a)treating a compound of formula (II) with an acid to obtain pantolactone; and 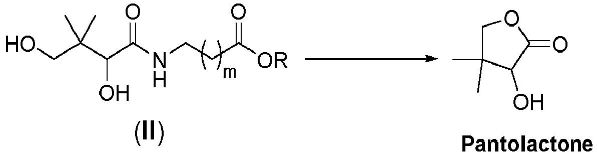
b) reacting the pantolactone with a compound of (III) to obtain the compound of formula (I), 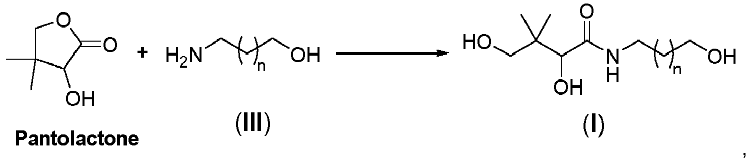
wherein R is selected from the group consisting of M or hydrogen or substituted or unsubstituted alkyl, wherein M is an alkali metal or earth alkali metal, particularly sodium, potassium, calcium, or magnesium, and each of m and n is independently an integer between 0-5.

2. The process of claim 1, wherein the compound of formula (II) is pantothenic acid or a salt thereof, more preferably pantothenic acid or the calcium salt thereof.

3. The process of claim 1, wherein the compound of formula (III) is 3-aminopropanol.

4. The process of any one of claims 1-3, wherein the compound of formula (II) is fermentatively produced.

5. The process of any one of claims 1-3, wherein the acid in the step a) is selected from any one or more of organic acids, inorganic acids and/or acidic cation exchange resins.

6. The process of claim 5, wherein the organic acids are selected from the group consisting of carboxylic acids such formic acid, acetic acid and trifluoroacetic acid, sulfonic acid or derivatives thereof such as methanesulfonic acid, tosic acid and trifluoromethanesulfonic acid; the inorganic acids are selected from the group consisting of hydrochloric acid, sulfuric acid, phosphoric acid and nitric acid; and the acidic cation exchange resins are polymeric organosulfonic acids such as Amberlyst 15.

7. The process of any one of claims 1-3, wherein the acid is selected from the group consisting of trifluoroacetic acid, hydrochloric acid, sulfuric acid, sulfonic acid, methanesulfonic acid, tosic acid, trifluoromethanesulfonic acid, phosphoric acid, nitric acid and Amberlyst 15, preferably from hydrochloric acid, sulfuric acid, methanesulfonic acid, nitric acid and Amberlyst 15, most preferably from methanesulfonic acid and sulfuric acid.

8. The process of any one of claims 1-7, wherein the acid in the step a) is added in an amount of from 0.01 mol to 20 moles, preferably from 0.1 moles to 10 moles, more preferably from 1 moles to 8 moles such as 1, 2, 3, 4, 5, 6 ,7 and 8 moles, per 1 mole of the compound of formula (II).

9. The process of any one of claims 1-3, wherein the reaction in the step a) is carried out at a temperature selected in the range of about 20°C to about 150°C, preferably of about 40°C to about 120°C, such as 80°C.

10. The process of any one of claims 1-9, wherein in the step a) the obtained pantolactone is separated from the produced by-products.

11. The process of any one of claims 1-9, wherein in the step a) the obtained pantolactone is separated from the produced by-products by extraction, and the extract or its concentrate containing pantolactone is used in the step b).

12. The process of any one of claim 10 or 11, wherein the produced by-products are metal salts such as calcium salts and amino acids such as beta-alanine and/or salts thereof.

13. The process of any one of claims 1-12, wherein the compound of formula (I) is so produced that at least about 95% such as about 97, 98, 99 or even 100% of the compound of formula (I) is present as (*R*)-configuration based on the total weight of the compound of formula (I).

14. A process for producing panthenol, comprising the step **i**) of fermentative production of pantothenic acid/pantothenate optionally followed by esterification thereof into pantothenic acid esters, and the step **ii**) of treating the pantothenic acid or salts and/or esters thereof obtained in the step **i**) with an acid to obtain pantolactone, and the step **iii**) of reacting the pantolactone obtained in the step **ii**) with 3-aminopropanol to obtain panthenol.

15. The process of claim 14, wherein the acid is selected from the group consisting of formic acid, acetic acid, trifluoroacetic acid, hydrochloric acid, sulfuric acid, sulfonic acid, methanesulfonic acid, tosic acid, trifluoromethanesulfonic acid, phosphoric acid, nitric acid and acidic cation exchange resins such as Amberlyst 15, preferably from hydrochloric acid, sulfuric acid, methanesulfonic acid, nitric acid and Amberlyst 15, most preferably from methanesulfonic acid and sulfuric acid.
